# EUROPEAN PATENT APPLICATION

(11) **EP 2 769 669 A1**
(43) Date of publication of application: **27.08.2014**
(21) Application number: 14156229.8
(22) Date of filing: 21.02.2014
(51) Int. Cl.: A61B 5/00, A61B 5/0245

(54) **Electronic device, heart-rate signal receiving method and program**

(30) Priority: 22.02.2013 JP 2013033600
(71) Applicant: Seiko Instruments Inc., Chiba-shi, Chiba (JP)
(72) Inventor: Koyama, Kazuhiro, Chiba-shi, Chiba (JP); Hasegawa, Takanori, Chiba-shi, Chiba (JP); Matsumoto, Ayumi, Chiba-shi, Chiba (JP); Takakura, Akira, Chiba-shi, Chiba (JP)
(74) Representative: Miller Sturt Kenyon

(57) **Abstract**

An electronic device to further reduce power consumption is provided. The electronic device includes a receiving circuit 280 which receives a heart rate signal transmitted from a heart rate measurement device 100, a noise detection unit 253 which determines whether the receiving circuit 280 normally receives the heart rate signal or not, and a receiving circuit control unit 256 which allows the receiving circuit 280 to be intermittently operated when the noise detection unit 253 determines that the heart rate signal is not received continuously for a given period of time.

## Description

### BACKGROUND OF THE INVENTION

### 1. Field of the Invention

The present invention relates to an electronic device, a heart-rate signal receiving method and a program.

### 2. Description of Related Art

A heart rate measurement system has been known from the past, which includes a heart rate measurement device detecting a heart rate and transmitting a signal corresponding to the detected heart rate by wireless, and an output device calculating the heart rate and so on based on the signal transmitted by wireless from the heart rate measurement device and outputting the heart rate and so on. Power consumption of the heart rate measurement system is high as wireless communication is performed between the heart rate measurement device and the output device. Additionally, it is difficult to mount a large battery on the output device when the output device is a small-sized device such as a wrist-watch type device. Therefore, it is required that power consumption of the output device is reduced. Accordingly, an output device is known, in which a receiving circuit is operated to start receiving a signal by a user's operation and the operation of the receiving circuit is stopped when a state of not receiving the signal continues for a given period of time to thereby reduce power consumption (for example, refer to JP-A-2007-28467 (Patent Document 1)).

Moreover, a pulse rate meter is known, which has an automatic power off function in which the power of a given portion is automatically shut off when a detection signal of a pulse rate sensor is not inputted, and executes the auto power off function only based on a pulse rate signal by determining the difference between the pulse rate signal and signals other than the pulse rate (for example, refer to JP-A-6-93884 (Patent Document 2)).

However, when the receiving circuit is operated to start receiving the signal by the user's operation in the known output device, the receiving circuit is operated until the state of not receiving the signal continues for a given period of time. Accordingly, for example, when the output device receives a noise signal, there is a problem that power is consumed as it is difficult to stop the receiving circuit. In the case where the technique described in Patent Document 2 is used for the receiving circuit, the automatic power off function is executed even when communication is temporarily interrupted, therefore, it is difficult to restart the communication. Accordingly, there is a problem that it is necessary to constantly supply power to the receiving circuit when performing communication and it is difficult to reduce power consumption of the output device.

### SUMMARY OF THE INVENTION

It is an aspect of the present application to provide an electronic device, a heart-rate signal receiving method and a program capable of further reducing power consumption.

According to an embodiment of the present application, there is provided an electronic device including a receiving circuit receiving a heart rate signal transmitted from an external device, a determination unit determining whether the receiving circuit normally receives the heart rate signal or not and a receiving circuit control unit allowing the receiving circuit to be intermittently operated when the determination unit determines that the heart rate signal is not normally received continuously for a given period of time.

In the electronic device according to the present application, the determination unit may determine whether the signal received by receiving circuit is the heart rate signal or a noise signal, determining that the receiving circuit normally receives the heart rate signal when determining that the signal received by the receiving circuit is the heart rate signal, and determining that the receiving circuit does not normally receive the heart rate signal when determining that the signal received by the receiving circuit is the noise signal.

The electronic device according to the present application may further include a time measurement unit measuring time, in which the receiving circuit control unit may allow the receiving circuit to be intermittently operated when the time measurement unit does not measure time continuously for a given period of time.

The electronic device according to the present application may further includes an input unit receiving an input, in which the receiving circuit control unit may allow the receiving circuit to be intermittently operated when the input unit does not receive the input continuously for a given period of time.

In the electronic device according the present application, the receiving circuit control unit may stop the operation of the receiving circuit when the receiving circuit is intermittently operated continuously for a given period of time.

In the electronic device according to the present application, the receiving circuit control unit may allow the receiving circuit to be normally operated in the case where the time measurement unit is during time measurement or the input unit receives the input as well as the determination unit determines that the receiving circuit normally receives the heart rate signal while the receiving circuit is intermittently operated.

Also according to the embodiment of the present application, there is provided a heart-rate signal receiving method including the steps of determining whether a receiving circuit normally receives a heart rate signal transmitted by an external device or not and allowing the receiving circuit to be intermittently operated when determination that the heart rate signal is not normally received continues for a given period of time in the step of determination.

Also according to the embodiment of the present application, there is provided a program for allowing a computer to execute the steps of determining whether a receiving circuit normally receives a heart rate signal transmitted by an external device or not and allowing the receiving circuit to be intermittently operated when determination that the heart rate signal is not normally received continues for a given period of time in the step of determination.

According to the present application, the receiving circuit receives the heart rate signal transmitted from the external device. The determination unit determines whether the receiving circuit normally receives the heart rate signal or not. The receiving circuit control unit allows the receiving circuit to be intermittently operated when the determination unit determines that the heart rate signal is not normally received continuously for a given period of time. Accordingly, it is possible to further reduce power consumption.

### BRIEF DESCRIPTION OF THE DRAWINGS

Embodiments of the present invention will now be described by way of further example only and with reference to the accompanying drawings, in which:
Fig. 1 is a block diagram showing a configuration of a heart rate measurement system according to an embodiment;
Fig. 2 is a view showing an outer appearance of a heart rate measurement device according to the embodiment;
Fig. 3 is a view showing an outer appearance of an output device according to the embodiment; and
Fig. 4 is a flowchart showing an operation procedure performed when the output device according to the embodiment receives the heart rate signal.

### DESCRIPTION OF EMBODIMENTS

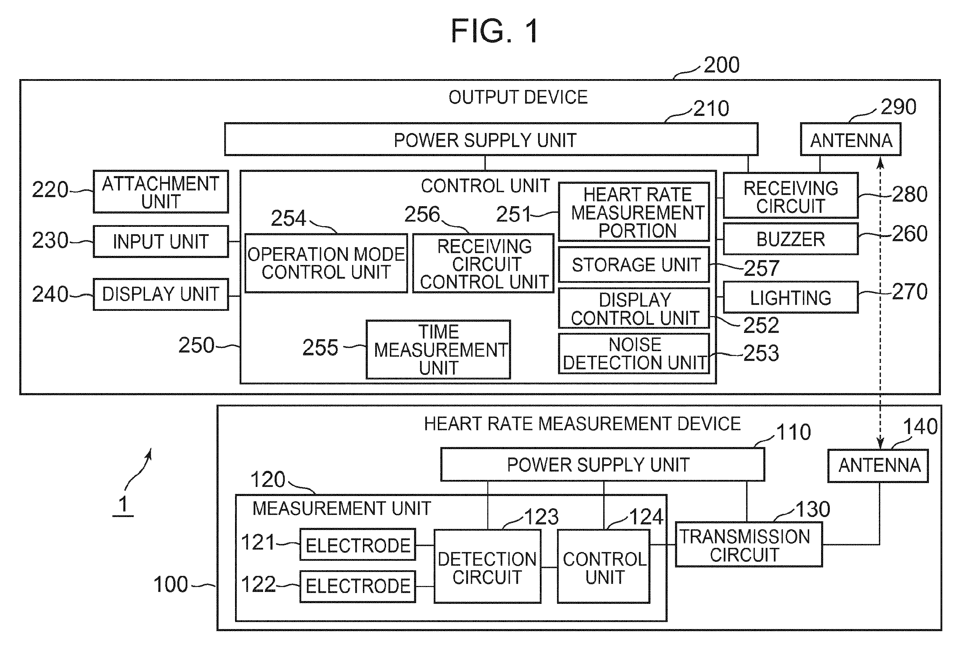

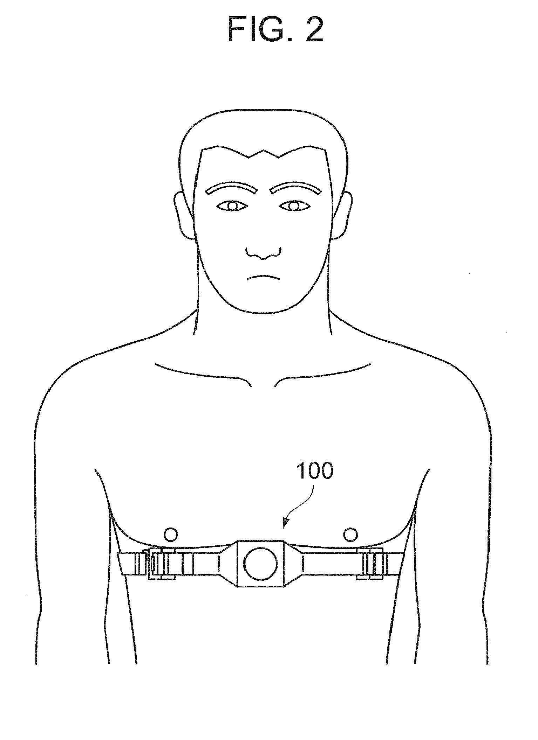

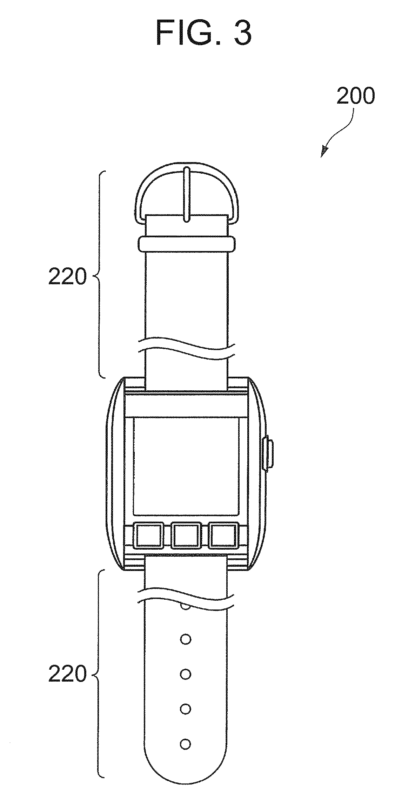

Hereinafter, an embodiment of the present invention will be explained with reference to the drawings. Fig. 1 is a block diagram showing a configuration of a heart rate measurement system 1 according to the embodiment. The heart rate measurement system 1 includes a heart rate measurement device 100 (external device) and an output device 200 (electronic device). Fig. 2 is a view showing an outer appearance of the heart rate measurement device 100 according to the embodiment. As shown in the drawing, the heart rate measurement device 100 is formed to have an approximately ring-shape so as to be attached over the entire circumference of a chest portion of a user. The heart rate measurement device 100 is attached to the chest portion of the user by using a band, detecting a heart rate signal generated by heart beats by allowing a pair of electrodes to contact the chest portion of a body (the surface of a living body) and transmitting the detected heart rate signal to the output device 200. Fig. 3 is a view showing an outer appearance of the output device 200 according to the embodiment. As shown in the drawing, the output device 200 is, for example, a wrist watch-type device having a stopwatch function, which is attached to a wrist (arm) of the user by using a band 220. The output device 200 is formed to have an approximately ring-shape so as to be attached over the entire circumference of a wrist of the user.

Hereinafter, the explanation will return to Fig. 1. The heart rate measurement device 100 includes a power supply unit 110, a measurement unit 120, a transmission circuit 130 and an antenna 140. The power supply unit 110 supplies electric power to respective units included in the heart rate measurement device 100. The measurement unit 120 includes an electrode 121, an electrode 122, a detection circuit 123 and a control unit 124, detecting a heart rate signal generated by heart beats and outputting the detected heart rate signal. The electrode 121 and the electrode 122 configure a pair of electrodes, detecting a heart rate signal generated by heart beats. The detection circuit 123 outputs the heart rate signal detected by the electrode 121 and the electrode 122 to the control unit 124.

The control unit 124 controls respective units included in the heart rate measurement device 100. For example, the control unit 124 transmits the inputted heart rate signal to the output device 200 through the transmission circuit 130 and the antenna 140. The transmission circuit 130 transmits the heart rate signal detected by the measurement unit 120 to the output device 200 through the antenna 140. The antenna 140 performs transmission/reception by wireless communication.

The output device 200 includes a power supply unit 210, an attachment unit 220, an input unit 230, a display unit 240, a control unit 250, a buzzer 260, a lighting 270, a receiving circuit 280 and an antenna 290. The power supply unit 210 supplies electric power to respective units included in the output unit 200 except the attachment unit 220.

The attachment unit 220 is a band for attaching the output device 200 to a user's arm. The input unit 230 receives an input of an operation instruction from a user and outputs the inputted instruction to the control unit 250. For example, the input unit 230 receives inputs of instructions for starting a measurement of time, for terminating the measurement of time and so on.

The display unit 240 is a display for displaying information. The control unit 250 controls respective units included in the output device 200. The control unit 250 includes a heart rate measurement portion 251, a display control unit 252, a noise detection unit 253 (determination unit), an operation mode control unit 254, a time measurement unit 255, a receiving circuit control unit 256 and a storage unit 257.

The heart rate measurement portion 251 calculates a heart rate in accordance with a pulse interval of the heart rate signal received from the heart rate measurement device 100 at fixed time intervals. Here, the pulse interval of the heart rate signal is, for example, an interval between peaks of the heart rate signal. The display control unit 252 displays the heart rate measured by the heart rate measurement portion 251, time measured by the time measurement unit 255 and the like on the display unit 240.

The noise detection unit 253 determines whether the receiving circuit 280 normally receives the heart rate signal or not. As a determination method, the noise detection unit 253 determines whether the signal received by the receiving circuit 280 is a heart rate signal or a noise signal. Then, the noise detection unit 253 determines that the receiving circuit 280 normally receives the heart rate signal when determining that the signal received by the receiving circuit 280 is the heart rate signal, and the noise detection unit 253 determines that the receiving circuit 280 does not normally receive the heart rate signal when determining that the signal received by the receiving circuit 280 is the noise signal.

For example, in the case where the heart rate measurement device 100 transmits the heart rate signal by using a pulse wave of 5KHz, the noise detection unit 253 determines whether the signal received by the receiving circuit 280 is the pulse wave or not by pattern matching. Then, the noise detection unit 253 determines that the signal received by the receiving circuit 280 is the heart rate signal when determining that the signal received by the receiving circuit 280 is the pulse wave. The noise detection unit 253 determines that the signal received by the receiving circuit 280 is the noise signal when determining that the signal received by the receiving circuit 280 is not the pulse wave. As noise signals, signals generated by a television, a stereo, a personal computer and so on can be cited.

The method of determining whether the receiving circuit 280 normally receives the heart rate signal performed by the noise detection unit 253 or not is not limited to the above method, and any other method may be used. Additionally, the method of determining whether the signal received by the receiving circuit 280 is the heart rate signal or the noise signal is not limited to the above method, and any other method may be used.

The operation mode control unit 254 changes an operation mode of the output device 200 based on the input received by the input unit 230. In the embodiment, the output device 200 operates in three types of operation modes, which are a clock mode, a chronograph mode and an alarm mode. The output device 200 displays the time on the display unit 240 when operating in the clock mode. The output device 200 also measures the time based on the input received by the input unit 230 and displays the measured time and the heart rate measured by the heart rate measurement portion 251 on the display unit 240 when operating in the chronograph mode. The output device 200 further sounds the buzzer 260 at the set time when operating in the alarm mode.

The time measurement unit 255 has an oscillator unit generating a clock used for clocking, executing the stopwatch function of measuring time. For example, the time measurement unit 255 starts the measurement of time when the input unit 230 receives an input for instructing "start clocking" and terminates the measurement of time when the input unit 230 receives an input for instructing "terminate clocking" while the operation mode of the output device 200 is the chronograph mode.

The receiving circuit control unit 256 controls the operation of the receiving circuit 280. For example, the receiving circuit control unit 256 allows the receiving circuit 280 to be normally operated when the operation mode control unit 254 changes the operation mode of the output device 200 to the chronograph mode. The receiving circuit control unit 256 stops the operation of the receiving circuit 280 when the operation mode control unit 254 changes the operation mode of the output device 200 to operation modes other than the chronograph mode (for example, the clock mode or the alarm mode).

The receiving circuit control unit 256 allows the receiving circuit 280 to be intermittently operated when the noise detection unit 253 determines that the signal received by the receiving circuit 280 is the noise signal continuously for a time A while the receiving circuit 280 is normally operated in the chronograph mode. The time A is, for example, one hour. Note that the time A may be previously fixed or may be arbitrarily set. The intermittent operation is an operation of repeating a receiving operation and a pause state. For example, the intermittent operation is an operation of alternately performing a receiving operation for 10 seconds and a pause state for 50 seconds. Periods of time of the receiving operation and the pause state in the intermittent operation may be previously fixed or may be arbitrarily set.

The receiving circuit control unit 256 allows the receiving circuit 280 to be intermittently operated when the state where the time is not measured (for example, a reset state or the pause state of time measurement) continues for a time B while the receiving circuit 280 is operated in the chronograph mode. The time B is, for example, one hour. Note that the time B may be previously fixed or may be arbitrarily set. The receiving circuit control unit 256 stops the operation of the receiving circuit 280 when the receiving circuit 280 is intermittently operated continuously for a time C. The time C is, for example, 12 hours. Note that the time C may be previously fixed or may be arbitrarily set.

The receiving circuit control unit 256 allows the receiving circuit 280 to be normally operated when the receiving circuit 280 receives the heart rate signal while the receiving circuit 280 is intermittently operated.

The storage unit 257 stores information used for operations of respective units included in the output device 200. The buzzer 260 is a speaker outputting sound. For example, the buzzer 260 outputs alarm sound and so on. The lighting 270 emits light to the display unit 240 so that the user can visually recognize the display unit 240 in a dark place and so on. The receiving circuit 280 receives signals transmitted from the outside (the heart rate signal and the noise signal) through the antenna 290. The antenna 290 performs transmission/reception by wireless communication.

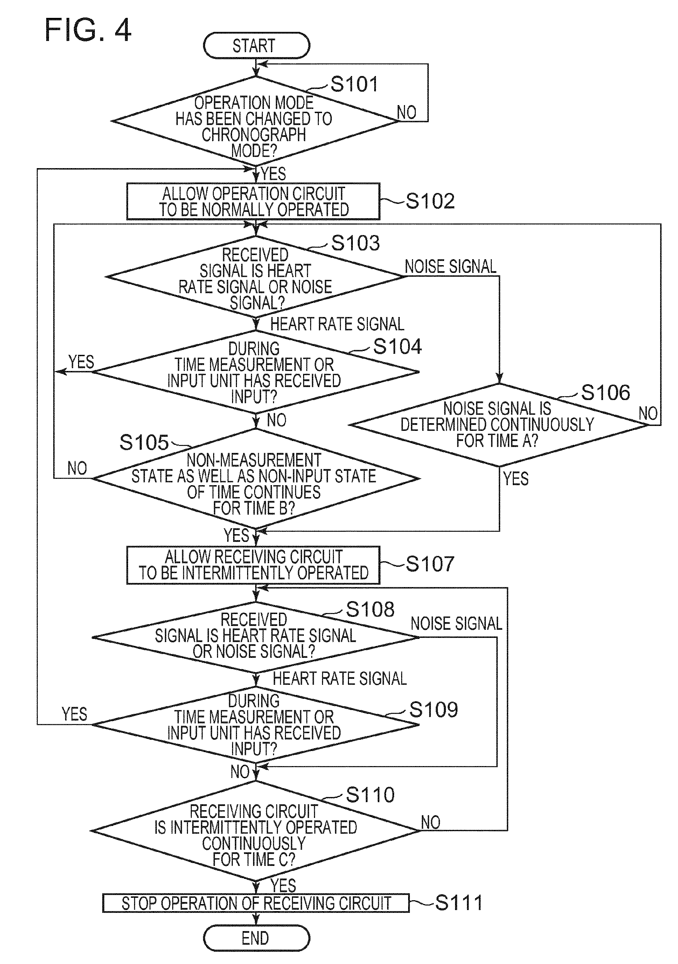

Next, an operation procedure performed when the output device 200 receives the heart rate signal will be explained. Fig. 4 is a flowchart showing the operation procedure performed when the output device 200 according to the embodiment receives the heart rate signal. In the embodiment, the user inputs an instruction for changing the operation mode to the chronograph mode to the input unit 230 when allowing the output device 200 to receive the heart rate signal.

(Step S101) The receiving circuit control unit 256 determines whether the operation mode control unit 254 has changed the operation mode of the output device 200 to the chronograph mode or not. When the receiving circuit control unit 256 determines that the operation mode has been changed to the chronograph mode, the process proceeds to Step S102, and the process of Step S101 is executed again in other cases.

(Step S102) The receiving circuit control unit 256 allows the receiving circuit 280 to be normally operated. The receiving circuit 280 starts to be operated and receives a signal. After that, the process proceeds to Step S103.

(Step S103) The noise detection unit 253 determines whether the signal received by the receiving unit 280 is the heart rate signal or the noise signal. When the noise detection unit 253 determines that the received signal is the heart rate signal, the process proceeds to Step S104, and when the noise detection unit 253 determines that the received signal is the noise signal, the process proceeds to Step S106.

(Step S104) The receiving circuit control unit 256 determines whether the time measurement unit 255 is during time measurement or whether the input unit 230 has received an input or not. When the receiving circuit control unit 256 determines that the time measurement unit 255 is during time measurement or that the input unit 230 has received an input, the process returns to Step S103, and the process proceeds to Step S105 in other cases.

(Step S105) The receiving circuit control unit 256 determines whether a state where the time measurement unit 255 does not measure time (non-measurement state) or a state where the input unit 230 does not receive an input (non-input state) continues for the time B (for example, one hour) or not. When the receiving circuit control unit 256 determines that the non-measurement state or the non-input state continues for the time B, the process proceeds to Step S107, and the process returns to Step S103 in other cases.

(Step S106) The receiving circuit control unit 256 determines whether the noise detection unit 253 determines that the received signal is the noise signal continuously for the time A (for example, one hour) or not. When the receiving circuit control unit 256 determines that the noise detection unit 253 determines that the received signal is the noise signal continuously for the time A, the process proceeds to Step S107, and the process returns to Step S103 in other cases.

(Step S107) The receiving circuit control unit 256 allows the receiving circuit 280 to be intermittently operated. After that, the process proceeds to Step S108.

(Step S108) The noise detection unit 253 determines whether the signal received by the receiving circuit 280 is the heart rate signal or the noise signal. When the noise detection unit 253 determines that the received signal is the heart rate signal, the process proceeds to Step S109, and when the noise detection unit 253 determines that the received signal is the noise signal, the process proceeds to Step S110.

(Step S109) The receiving circuit control unit 256 determines whether the time measurement unit 255 is during time measurement or whether the input unit 230 has received an input or not. When the receiving circuit control unit 256 determines that the time measurement unit 255 is during time measurement or that the input unit 230 has received an input, the process returns to Step S102, and the process proceeds to Step S110 in other cases.

(Step S110) The receiving circuit control unit 256 determines whether the receiving circuit 280 has been intermittently operated continuously for the time C (for example, 12 hours). When the receiving circuit control unit 256 determines that the receiving circuit 280 has been intermittently operated

continuously for the time C, the process proceeds to Step S111, and the process returns to Step S108 in other cases.

(Step S111) The receiving circuit control unit 256 stops the operation of the receiving circuit 280. After that, the process is terminated.

As described above, according to the embodiment, the receiving circuit control unit 256 changes the operation of the receiving circuit 280 to the intermittent operation in the case where the receiving circuit 280 receives the noise signal continuously for the time A even when the receiving circuit 280 receives the signal. The receiving circuit control unit 256 stops the operation of the receiving circuit 280 in the case where the receiving circuit 280 is intermittently operated continuously for the time C. Accordingly, power consumption of the output device 200 can be further reduced.

For example, even if the noise signal is received in the case where the operation mode of the output device 200 is maintained in the chronograph mode after the measurement of the heart rate is terminated, the operation of the receiving circuit 280 is allowed to be intermittently operated when the noise signal is received continuously for the time A. Furthermore, the receiving circuit control unit 256 stops the operation of the receiving circuit 280 when the receiving circuit 280 is allowed to be operated intermittently continuously for the time C. Accordingly, power consumption of the output device 200 can be further reduced even when the receiving circuit 280 receives the noise signal.

Also in the embodiment, when the noise signal is received continuously for the time A, the receiving circuit control unit 256 does not stop the operation of the receiving circuit 280 immediately but allows the receiving circuit 280 to be intermittently operated. Accordingly, the receiving circuit control unit 256 can allow the receiving circuit 280 to be normally operated when the receiving circuit 280 restarts receiving the heart rate signal, which further improves operability.

Also in the embodiment, the receiving circuit 280 is operated only in the case where the operation mode is the chronograph mode, and the operation of the receiving 280 is stopped in other operation modes, therefore, power consumption of the output device 200 can be further reduced.

All the functions of respective units included in the heart rate measurement device 100 according to the embodiment or part of them as well as all the functions of respective units included in the output device 200 or part of them can be realized by recording a program for realizing these functions in a computer readable recording medium and by reading the program recorded in the recording medium into a computer system to execute the program. The "computer system" referred to here includes OS and hardware such as peripheral devices.

Additionally, the "computer readable recording media" include portable media such as a flexible disk, a magneto-optical disk, a ROM or a CD-ROM and storage units such as a hard disk built in the computer system. The "computer readable recording media" may further include media holding the program dynamically for a short period of time such as networks including the Internet and a communication line used when transmitting the program through a communication line such as a telephone line, as well as media holding the program for a fixed period of time such as a volatile memory inside the computer system to be a server or a client in such case. The program may be a program for realizing part of the above described functions and may be a program which can realize the above functions by combination with a program already recorded in the computer system.

The embodiment of the present invention has been explained with reference to the drawings as described above, but the specific configuration is not limited to the embodiment, and design and the like within a scope of the invention as defined by the claims are also included.

For example, it may be possible to carry out heart rate measurement when the output device 200 is in a mode other than the chronograph mode.

## Claims

1. An electronic device (200) comprising:
a receiving circuit (280) for receiving a heart rate signal transmitted from an external device (100);
a determination unit (253) for determining whether the receiving circuit normally receives the heart rate signal or not; and
a receiving circuit control unit (256) for allowing the receiving circuit to be intermittently operated when the determination unit determines that the heart rate signal is not normally received continuously for a given period of time (A).

2. The electronic device according to claim 1,
wherein the determination unit is configured to determine whether the signal received by receiving circuit is the heart rate signal or a noise signal, determine that the receiving circuit normally receives the heart rate signal when determining that the signal received by the receiving circuit is the heart rate signal, and determine that the receiving circuit does not normally receive the heart rate signal when determining that the signal received by the receiving circuit is the noise signal.

3. The electronic device according to claim 1 or 2, further comprising:
a time measurement unit (255) for measuring time,
wherein the receiving circuit control unit is configured to allow the receiving circuit to be intermittently operated when the time measurement unit does not measure time continuously for a given period of time (B).

4. The electronic device according to any one of claims 1 to 3, further comprising:
an input unit (230) for receiving an input,
wherein the receiving circuit control unit is configured to allow the receiving circuit to be intermittently operated when the input unit does not receive the input continuously for a given period of time (B).

5. The electronic device according to any one of claims 1 to 4,
wherein the receiving circuit control unit is configured to stop the operation of the receiving circuit when the receiving circuit is intermittently operated continuously for a given period of time (C).

6. The electronic device according to any one of claims 1 to 5,
wherein the receiving circuit control unit is configured to allow the receiving circuit to be normally operated in the case where the time measurement unit is during time measurement or the input unit receives the input as well as the determination unit determines that the receiving circuit normally receives the heart rate signal while the receiving circuit is intermittently operated.

7. A heart-rate signal receiving method comprising the steps of:
(S103) determining whether a receiving circuit (280) normally receives a heart rate signal transmitted by an external device (100) or not; and
(S107) allowing the receiving circuit to be intermittently operated when determination that the heart rate signal is not normally received continues for a given period of time (A) in the step of determination.

8. A program for allowing a computer to execute the steps of:
(S103) determining whether a receiving circuit (280) normally receives a heart rate signal transmitted by an external device or not; and
(S107) allowing the receiving circuit to be intermittently operated when determination that the heart rate signal is not normally received continues for a given period of time (A) in the step of determination.
